# EUROPEAN PATENT SPECIFICATION

(11) **EP 3 172 207 B1**
(45) Date of publication and mention of the grant of the patent: **06.03.2019**
(21) Application number: 15739630.0
(22) Date of filing: 23.07.2015
(51) Int. Cl.: C07D 471/14, C07D 461/00

(54) **A PROCESS FOR THE PREPARATION OF CGMP-PHOSPHODIESTERASE INHIBITOR AND ORAL PHARMACEUTICAL FORMULATION COMPRISING TADALAFIL CO-PRECIPITATES**
VERFAHREN ZUR HERSTELLUNG DES CGMP-PHOSPHODIESTERASEHEMMERS UND ORALE PHARMAZEUTISCHE FORMULIERUNG MIT TADALAFIL-CO-PRÄZIPITATEN
PROCÉDÉ DE PRÉPARATION D'INHIBITEUR DE CGMP-PHOSPHODIESTÉRASE ET FORMULATION PHARMACEUTIQUE ORALE COMPRENANT DES CO-PRÉCIPITÉS DE TADALAFIL

(30) Priority: 23.07.2014 SI 201400267
(43) Date of publication of application: 31.05.2017
(60) Divisional application: 18205889.1
(73) Proprietor: KRKA, d.d., Novo mesto, 8000 Novo mesto (SI)
(72) Inventor: BARIC, Matej, 8322 Stopice (SI); BENKIC, Primoz, 1000 Ljubljana (SI); BOMBEK, Sergeja, 8000 Novo mesto (SI); KRASOVEC, Dusan, 8296 Krmelj (SI); SKRABANJA, Vida, 8000 Novo mesto (SI); VRECER, Franc, 8351 Straza pri Novem mestu (SI); BUKOVEC, Polona, 8000 Novo mesto (SI); HUDOVORNIK, Grega, 8000 Novo mesto (SI); KROSELJ, Vesna, 8000 Novo mesto (SI)
(74) Representative: Hoffmann Eitle
(86) International application number: PCT/EP2015/066871
(87) International publication number: WO 2016/012539

(56) References cited:
- EP-A1- 0 828 479
- WO-A1-2004/011463
- US-A1- 2012 189 694

## Description

### FIELD OF THE INVENTION

The present invention relates to a process for the preparation of CGMP-phosphodiesterase inhibitor, particularly tadalafil.

### BACKGROUND OF THE INVENTION

Tadalafil, chemically known as (6R-trans)-6-(1,3-benzodioxol-5-il)-2,3,6,7,12,12a-hexahydro-2-methyl-pyrazino[1', 2':1,6]pyrido[3,4-b]indole-1,4-dione, is a potent and selective inhibitor of the cyclic guanosine monophosphate (cGMP) - specific phosphodiesterase enzyme PDE5. It is shown below as structural formula I:

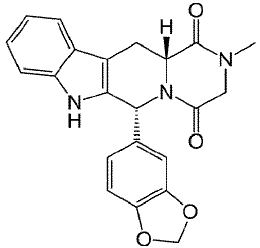

Tadalafil is marketed under the tradename CIALIS® and is used for the treatment of erectile dysfunction. The product is available as a film-coated tablet for oral administration containing 2.5, 5, 10 and 20 mg of active ingredient and the following inactive ingredients: lactose monohydrate, hydroxypropylcellulose, sodium lauryl sulfate, croscarmellose sodium, microcrystalline cellulose, magnesium stearate, hypromellose, triacetin, titanium dioxide (E171), iron oxide (E172) and talc.

Tadalafil is practically insoluble in water and very slightly soluble in organic solvent such as ethanol, methanol and acetone.

Problems associated with low solubility of tadalafil in ethanol and most of other organic solvents resulted in the need of large quantities of solvents required to perform synthesis and crystallization of tadalafil at industrial scale, which have unwanted technological, environmental and economical impact.

US Patent No. 5 859 006 describes the synthesis of the tadalafil and its intermediate (A) which involves reacting D-tryptophan methyl ester with a piperonal in the presence of dichloromethane and trifluoroacetic acid which provides a mixture of desired cis and undesired trans isomer of intermediate A with poor selectivity. The isomers are further separated by column chromatography. The cis isomer is further reacted with chloroacetyl chloride in chloroform, providing another intermediate of tadalafil (B) which reacted with methylamine to give tadalafil of formula (1) in methanol slurry requiring an additional purification step by flash chromatography.

An improved process in the synthesis of tadalafil via modified Pictet-Spengler reaction is described in WO 04/011463 in which D-tryptophan methyl ester hydrochloride and piperonal are condensed in anhydrous isopropyl alcohol to provide hydrochloride of intermediate A. After isolation of desirable cis isomer, the product is further reacted with chloroacetyl chloride and then with methylamine in THF to give tadalafil.

Therefore there still exists a need for an improved process for a synthesis and purification of tadalafil, which would overcome the disadvantages of the prior art processes.

### Summary of the invention

The above problem is solved by the manufacturing process characterized in appended claim 1. Preferred embodiments of this aspect of the present invention are specified in appended claims 2 to 4.

### DESCRIPTION OF THE INVENTION

The present invention provides technological steps to improve tadalafil synthesis and crystallization and/or purification process that have important technological, environmental and economical benefits.

In the present invention we have discovered that the aforementioned disadvantage of tadalafil can be overcome by conducting chemical reaction and crystallization step of tadalafil at increased pressure with surprisingly good efficiency and yield.

The present invention provides a process for the synthesis of tadalafil from D-tryptophan or a salt thereof or an ester of D-tryptophan with R-OH or a salt thereof, wherein R is as defined below, and piperonal via intermediates A and B to the final active substance with only one isolation step. In the following, the term "D-tryptophan derivative" is sometimes used to characterize the above-mentioned starting material "D-tryptophan or a salt thereof or an ester of D-tryptophan with R-OH or a salt thereof". Reaction steps are performed at elevated temperature and pressure in organic solvents or in the mixture thereof or in the mixture thereof with water. Preferably, the process is performed in a closed reaction vessel at and above the reflux temperature of the solvent or solvents' mixture and under increased pressure of about 0.1MPa to 0.6MPa, preferably about 0.1 to about 0.4MPa. The advantages of these conditions are faster reaction times, high selectivity, reduced quantities of organic solvents and lower costs of production.

It is the one of the great accomplishments of the present inventors to have found that the reaction of interest is robust with respect to temperature increase so that the above-mentioned benefits may be accomplished (without concomitant increase of undesired side reactions) if the reaction temperature is increased to a temperature above the boiling point of the employed solvent or solvent mixture at normal atmospheric pressure while keeping the reaction mixture in a liquid state. In the context of the present invention, the term "normal atmospheric pressure" is meant to indicate a pressure of 1013.25 hPa.

According to one aspect of the invention, it is therefore essential to carry out the first step of the synthesis of tadalafil from D-tryptophan or a salt thereof or an ester of D-tryptophan with R-OH or a salt thereof, wherein R is as defined below, and piperonal via intermediates A and B, as specified below, at a temperature that is above the boiling point of the employed solvent or employed solvent mixture at atmospheric pressure. This is accomplished by carrying out the reaction in a closed vessel. The reaction temperature thus may be increased by the resulting increase of pressure and associated increase of boiling point of the solvent or solvent mixture inside the reaction vessel. If desired, it is even possible to further increase the reaction temperature inside of the reaction vessel by injecting nitrogen (or other suitable) gas into the reaction vessel to thereby further increase the internal pressure and associated boiling point of the solvent or solvent mixture.

Consequently, according to this embodiment of the present invention, the internal pressure inside the reaction vessel is chosen such that the boiling point of the solvent or solvent mixture and therefore the reaction temperature during the first reaction step is from 2 to 100°C, preferably from 5 to 50°C more preferably from 10 to 30°C, above the respective boiling point of the solvent or solvent mixture at normal atmospheric pressure. The applied internal pressure is not particularly limited as far as it is sufficiently high to permit increasing the reaction temperature during the first step of the process to a desired temperature within the above identified ranges while keeping the reaction mixture in a predominantly liquid state. The upper limit of the applied internal pressure is not limited by the scope of the present invention. However, for practical and cost reasons, it may be advantageous to not exceed 0.6MPa. In this manner, the use of expensive specialized equipment for handling very high pressures can be avoided.

By the process of the present invention the quantity of organic solvent or a mixture thereof or a mixture of the solvent with water in the synthesis and/or purification step of tadalafil is reduced for 1 to 8 times, preferably for 5 times in comparison to the prior art processes. Prior art processes for example describes crystallization from 2-propanol in quantity of 200L per 1kg of active substances. According to the present invention the crystallization from organic solvents or the mixture thereof with addition of water as antisolvent is preferred, since the content of the solvent required for successful crystallization is substantially reduced. We have surprisingly discovered that tadalafil is soluble in 1 to 8 times reduced quantity of solvent when water is added to the mixture and the mixture is heated above the reflux temperature. The volume ratio of the organic solvent to water may vary from 99:1 to 50:50, preferably 80:20 to 60:40. The amount of the solvent or the solvent mixture used for crystallization of 1kg of tadalafil may vary from 20L to 100L, preferably from 30L to 50L.

The term "reflux temperature" refers to the temperature of the boiling point of the reaction mixture at the pressure employed in the reaction vessel.

Preferred organic solvents used for high pressure reaction crystallization may be selected from the group of nitriles, aromatic hydrocarbons, aliphatic alcohols, alkyl esters, ketones, cyclic ethers, mixture thereof and mixture thereof with water. The term "nitriles" refers to organic compounds having a -CN functional group, among them preferably acetonitrile is used. The term "aromatic hydrocarbons" refers to C₆-C₁₀ monocyclic and polycyclic aromatic or substituted hydrocarbons including benzene, toluene, tetrahydronaphtalene, optionally having a C₆-C₁₂ alkyl, halo, or nitro substituent and a mixture of these, among them toluene is preferred. The term "alcohol" refers to organic compounds having the general structure R-OH, wherein R is a linear or branched alkyl group, among them preferably 2-propanol is used. The term "alkyl esters" as used herein, refers to organic compounds having the general structure R-COOR', wherein R and R' are branched or linear alkyl groups. The term "ketones" as used herein, refers to acetone, methyl ethyl ketone, diethyl ketone methyl propyl ketone, t-butyl methyl ketone. The term "cyclic ethers" as used herein, refers preferably to tetrahydrofuran, dioxane. 2-propanol and mixture thereof with water is the most preferred solvent for use in the process of the present invention. The ratio of 2-propanol to water in a solvent mixture thereof may vary from 99:1 to 50:50, preferably from 80:20 to 60:40, most preferably from 75:25 to 65:35.

Most preferably the process for preparing tadalafil according to the present invention comprises the following steps:
(i) suspending D-tryptophan or a salt thereof or an ester of D-tryptophan with R-OH or a salt thereof, wherein R is C₁-C₆ alkyl and especially Me, Et, Pr, iPr, tBu, cyclohexyl, more preferably Me, and piperonal in an organic solvent and heating the suspension in a closed reaction vessel above the reflux temperature and at pressure of at least 0.1MPa or heating the suspension in the closed reaction vessel to a temperature above the boiling point at normal atmospheric pressure of the employed solvent or solvent mixture. This reaction is carried out under acidic conditions. An acid HX, which is an organic or inorganic acid and preferably HCl, may be used for acidifying the reaction mixture. Alternatively, it is possible to use the starting material D-tryptophan derivative, as specified above, in the form of a salt with HX, preferably HCl. A 1:1 molar ratio of starting material D-tryptophan or ester thereof to HX is therefore a preferred embodiment. However, other molar ratios within the range of 1:100 to 5:1 and preferably 1:10 to 2:1 are also suitable for practicing the present invention. Similar considerations apply regarding the molar ratio of starting materials D-tryptophan derivative:piperonal. This molar ratio is preferably around 1:1 but other ratios within the range of 2:1 to 1:2 and preferably 1.5:1 to 1:1.5 and most preferably 1.2:1 to 1:1.2 may be used. Typical reaction times (i.e. durations of step (i)) are between 1 and 24 hours, preferably 1.5 to 5 hours and more preferably 2 to 3 hours. This means that the reaction time is considerably shortened in comparison with state of the art processes typically requiring a reaction time of two or more days.
(ii) Chloroacylation of the "in situ" formed intermediate. It is assumed that this intermediate is intermediate A which is a mixture of cis and trans isomer, with the following formula, wherein R is H, or C₁-C₆ alkyl and especially, Me, Et, Pr, *i*Pr, *t*Bu, cyclohexyl, preferably Me, and HX is an organic or inorganic acid, preferably HCI to indicate that the intermediate A is mostly present in the reaction mixture in ionized (protonated) form, i.e. that in the form of a dissolved salt.

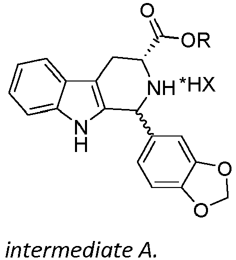

In a preferred embodiment, it is assumed that intermediate A in the reaction mixture is characterized by the following structure:

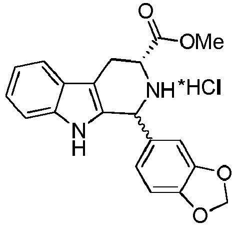

The chloroacylation reaction is carried out after the first step of reaction in the mixture at a temperature typically below 25°C preferably between 0°C and 15°C. The typically used reagent combination is chloroacetyl chloride in the presence of an organic or inorganic base preferably an alkali metal carbonate or an alkaline earth metal carbonate. The molar ratio between chloroacetyl chloride used in chloroacetylation reaction and D-tryptophan derivative used in step (i) is preferably within a range 5:1 to 1:1, preferably 2.5:1 to 1:1 and most preferably 2:1 to 1.2: 1.
(iii) Subsequently, the process includes isolation of the obtained intermediate B, which has the following formula, where R is H, or C₁-C₆ alkyl and especially Me, Et, Pr, *i*Pr, *t*Bu, cyclohexyl, preferably Me 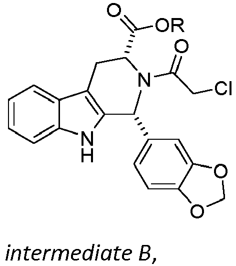 and converting it to tadalafil via cyclisation with methylamine in the mixture of organic solvent with water, in a closed reaction vessel at temperature above the reflux temperature and at pressure of at least 0.1MPa, or according to the other embodiment, in a closed vessel at an increased internal pressure and at a temperature above the boiling point of the employed solvent at normal atmospheric pressure,
(iv) precipitation and isolation of the product,
(v) optionally performing re-crystallization under increased pressure.

The organic solvent used in step (i) and step (ii) is preferably selected from nitriles, aromatic hydrocarbons, aliphatic alcohols, alkyl esters, ketones, cyclic ethers, mixture thereof and mixture thereof with water. The term "nitriles" refers to organic compounds having a -CN functional group, among them preferably acetonitrile is used. The term "aromatic hydrocarbons" refers to C6-C10 monocyclic and polycyclic aromatic or substituted hydrocarbons including benzene, toluene, tetrahydronaphtalene, optionally having a C6-C12 alkyl, halo, or nitro substituent and a mixture of these, among them toluene is preferred. The term "alcohol" refers to organic compounds having the general structure R-OH, wherein R is a linear or branched alkyl group, among them preferably 2-propanol is used. The term "alkyl esters" as used herein, refers to organic compounds having the general structure R-COOR', wherein R and R' are branched or linear alkyl groups. The term "ketones" as used herein, refers to acetone, methyl ethyl ketone, diethyl ketone methyl propyl ketone, t-butyl methyl ketone. The term "cyclic ethers" as used herein, refers preferably to tetrahydrofuran, and dioxane. Acetonitrile is the most preferred solvent for use in steps (i) and (ii) of the process of the present invention.

The organic solvent used in step (iii), (iv) and step (v) is preferably selected from nitriles, aromatic hydrocarbons, aliphatic alcohols, alkyl esters, ketones, cyclic ethers, mixture thereof and mixture thereof with water, preferably a mixture of alcohol and water.

The organic solvent used in step (iii), step (iv) and step (v) is preferably alcohol such as ethanol, methanol, n-propanol, 2-propanol, most preferably 2-propanol. According to a preferred embodiment of the present invention, the above-mentioned organic solvent is used in combination with water. While water normally functions as an anti-solvent for tadalafil, the present inventors have surprisingly found that adding minor quantities of water permits to accomplish an even greater (steeper) increase of solubility of tadalafil when increasing the temperature of the solvent as compared to the increase of solubility when increasing the temperature of the solvent in the absence of water. Generally, the relative amount of water to be added may be selected in the range of 1 to 50 wt.%, preferably 5 to 30 wt.%, based on the weight of the organic solvent. However, it is advisable to adjust and optimize the chosen amount of water with respect to the specific organic solvent that is employed. This can be easily done by carrying out a series of dissolution experiments, wherein different amounts of water are added (e.g. in steps of 5 wt.% based on the weight of the organic solvent) and wherein the amount of dissolved tadalafil at the temperature of interest is determined.

The pressure used in step (i) and/or in step (iii) is preferably in the range of 0.1MPa to 0.6MPa, most preferably 0.1MPa to 0.4MPa. It is also preferred to apply a pressure that is at least slightly above normal atmospheric pressure, for instance a pressure in the range of 0.11 MPa to 0.6 MPa or most preferably 0.12 MPa to 0.6 MPa. According to the above-mentioned other embodiment, the pressure inside the reaction vessel is not particularly limited as far as it is sufficiently high to ensure that the boiling point of the employed solvent or solvent mixture is above the corresponding boiling point of the employed solvent or solvent mixture at normal atmospheric pressure. The above-mentioned pressure ranges of 0.11 MPa to 0.6 MPa or most preferably 0.12 MPa to 0.6 MPa may also be considered in this embodiment.

Any reaction vessel can be used provided that it can withstand the chosen pressure. For instance, a standard overpressure reactor, typically usable to pressures up to 0.6 MPa, can be used.

It is a characteristic feature and benefit of the process of the present invention that it is advantageously not necessary to physically separate and remove undesired diastereomer in step (i). Hence, this step is absent from the process of the present invention.

The process for preparing tadalafil according to a preferred embodiment of the present invention is disclosed in Scheme 1.

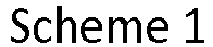

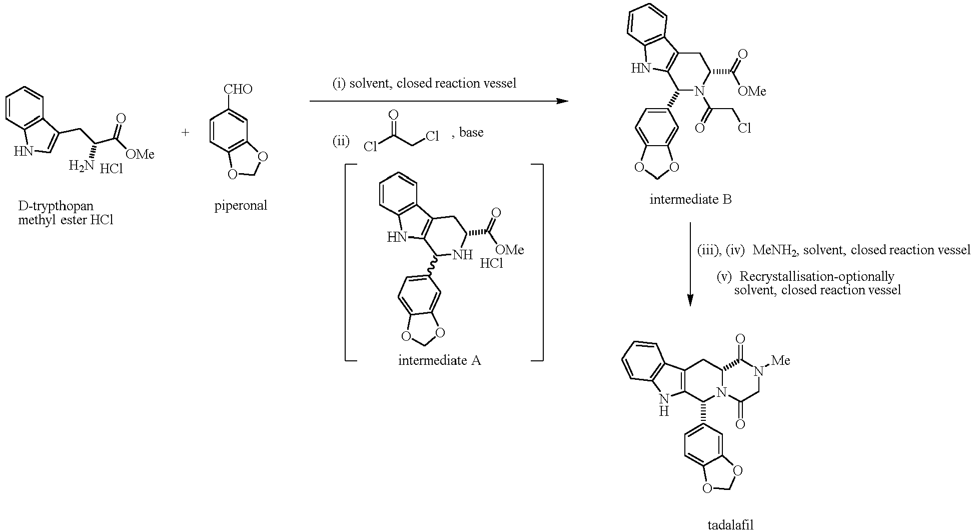

By the process of the present invention a highly pure product is provided with the assay of at least 99.5% wt. The product is substantially free of impurities, either organic or inorganic impurities. The high purity of the product is characterized by that that the content of any of the individual impurities is below 0.20%, preferably below 0.15% and most preferably below 0.10%.

The selected examples illustrate the present invention without limiting the scope of the invention.

### EXAMPLES

### Methods

The chemical purity of tadalafil is assessed by high pressure liquid chromatography (HPLC) with a column of the type Zorbax SB-C8 250 x 4.6 mm i.d., 5 µm particles; column temperature: 40°C; detector: UV 220 nm; flow rate: 0.7 ml/min; injection volume: 5 µL; mobile phase: A: 0.01M phosphate buffer solution pH 2; B: acetonitrile/methanol = 80/20 (V/V); Gradient (applicable for analysis of chromatographic purity): 0'=15% B, 7.5'=40% B, 14'=60% B, 20'-22'=90% B, 23'-28'=95% B, 30'-33'=15% B; Isocratic separation (applicable for analysis of assay): 40% of mobile phase A and 60% of mobile phase B.

This HPLC method is generally applicable for the analysis of tadalafil, chromatographic purity and assay.

### Sample preparation:

Chromatographic purity: The sample solution is prepared in concentration about 0.25 mg/mL. Sonication is applied during dissolving.

Dilution solvent is 90% methanol.

Assay: The sample solution is prepared in concentration of about 0.1 mg/mL. Sonication is applied during dissolving.

### Calculation:

Chromatographic purity: Area per cent method was used, solvent peaks were not integrated. Assay: External standard method was used.

Unless otherwise specified, dissolution profiles were obtained by testing samples containing 20mg of tadalafil in 900mL of an aqueous solution of 0.1M HCl + 0.2% sodium lauryl sulfate at 37°C, using USP Apparatus I with baskets rotating at a speed of 100 rpm and automatic UV detection.

Unless otherwise specified, the tadalafil co-precipitate used in the examples has average particle size from 10 to 60 µm. The term "average particle size" as used herein refers to the volume mean diameter of particles. The diameter and volume mean diameter can be determined by laser light scattering using preferably a Malvern-Mastersizer Apparatus MS 2000 and a Scirocco dispersion cell (dry dispersion).

Tablet hardness was measured by determining lateral breaking strength using a KRAEMER (UTS) system.

Specific surface area of tadalafil and tadalafil co-precipitate s was determined by a gas sorption system based on the nitrogen adsorption, using the 6-point Brunauer, Emmett and Teller (BET) method. Prior to the analysis the sample was degassed for 2 hours under vacuum

### Example 1: Synthesis of tadalafil intermediate B via intermediate A

D-tryptophan methyl ester hydrochloride (9g) and piperonal (6g) was suspended in acetonitrile (60mL). The reaction mixture was stirred and heated at about 105°C for three to five hours in an autoclave. The reaction suspension was cooled to ambient temperature and aqueous solution (60mL) of sodium carbonate (4.1g) was added. The mixture was then cooled in an ice bath and the solution of chloroacetyl chloride (5.1mL) in acetonitrile was slowly added to the reaction mixture. A solid was obtained, filtered and washed twice with aqueous solution of acetonitrile. The crude product was dried, and intermediate B (13.4g) with a purity of 97% (HPLC area%) was obtained.

### Example 1A:

D-tryptophan methyl ester hydrochloride (8.2kg) and piperonal (5.1kg) was suspended in acetonitrile (55L). The reaction mixture was stirred and heated at about to 105°C for three hours in the reactor vessel. The reaction suspension was cooled to ambient temperature and aqueous solution (55L) of sodium carbonate (4.8kg) was added. The mixture was then cooled in an ice bath and the solution of chloroacetyl chloride (5.2L) was slowly added to the reaction mixture at 5-10°C. A solid was obtained, centrifuged and washed twice with aqueous solution of acetonitrile (2x 12L). The crude product was dried at temperature up to 50°C, and intermediate B (12.3kg) with a purity of 98% (HPLC area%) was obtained.

### Comparative example 1:

D-tryptophan methyl ester hydrochloride (9.0g) and piperonal (5.84g) was suspended in acetonitrile (60mL). The reaction mixture was stirred and heated at about to 80-85°C for 15-20 hours in the reactor vessel. The reaction suspension was cooled to 0-10°C. The Intermediate A was then isolated on centrifuge and was dried at temperature up to 60°C.
The isolated dried Intermediate A (12,8g) was charged into reactor and suspended with ethyl acetate. The aqueous solution (60mL) of sodium carbonate (5.3g) was added to precooled suspension of Intermediate A. The chloroacetyl chloride (3.4mL) was slowly added to the above reaction mixture. The solid was obtained, centrifuge and washed twice with water (2x 10mL). The crude product was dried at temperature up to 70°C, and intermediate B (11.8g) with a purity of 99% (HPLC area%) was obtained.

### Example 2: Synthesis of tadalafil

Intermediate B (4g) obtained in Example 1 and 40% aqueous methylamine solution (1.6mL) were dissolved in 70% aqueous solution of 2-propanol (120mL) while heating in a closed reaction vessel above the reflux temperature (110-120°C) for two to five hours. The solution was hot filtered and cooled on an ice bath. The precipitated product was filtered and dried. The purity of the product was 99.9% (HPLC area%) and the particle distribution of the product was D(90) of about 144 microns.

### Example 2A: Synthesis of tadalafil

Intermediate B (12.3kg) obtained in Example 1A and 40% aqueous methylamine solution (4.76L) were dissolved in 70% aqueous solution of 2-propanol (402L) while heating in a closed reaction vessel above the reflux temperature (110-120°C) for three hours. The solution was hot filtered and cooled on an ice bath. The precipitated product was filtered and dried. The final product (9.8kg) with a purity of more than 99.99% (HPLC area%) and the particle distribution of the product was D(90) of about 155 microns was obtained.

### Comparative example 2:

Intermediate B (10g) obtained in the above comparative example 1 and 31% ethanolic methylamine solution (12.3mL) were suspended in absolute ethanol (150mL). The suspension was heated up to 55°C for 3 - 6 hours. The suspension was cooled on an ice bath. The product was filtered and dried. The crude product (8.22g) with a purity of more than 99.9% (HPLC area%) was obtained and crystallized from hot DMSO solution. The product is crystallized with addition of water.

### Example 3: Recrystallization of tadalafil

Tadalafil (700g) (99% purity) was suspended in 70% aqueous solution of 2-propanol (24.6L) and suspension was heated to about 110°C in an autoclave at pressure of 0.31MPa until the material was dissolved. The obtained solution was then hot filtrated and cooled to about 10°C. The isolated tadalafil (660g) has a purity of 99.95% (HPLC area%) and the particle distribution D(90) of about 144 microns.

### Example 3A: Recrystallization of tadalafil

Tadalafil (5g) (99% purity) was suspended in 70% aqueous solution of acetone (100mL) and suspension was heated to about 90°C in an autoclave at pressure of 0.28MPa until the material was dissolved. The obtained solution was then hot filtrated and cooled to about 10°C. The isolated tadalafil (4.44g) has a purity of 99.99% (HPLC area%).

### Example 3B: Recrystallization of tadalafil

Tadalafil (4g) (99% purity) was suspended in 70% aqueous solution of acetonitrile (100mL) and suspension was heated to about 85°C in an autoclave at pressure of 0.2MPa until the material was dissolved. The obtained solution was then hot filtrated and cooled to about 10°C. The isolated tadalafil (3g) has a purity of 99.99% (HPLC area%).

### Example 3C: Recrystallization of tadalafil

Tadalafil (5g) (99% purity) was suspended in 70% aqueous solution of tetrahydrofuran (60mL) and suspension was heated to about 120°C in an autoclave at pressure of 0.3MPa until the material was dissolved. The obtained solution was then hot filtrated and cooled to about 10°C. The isolated tadalafil has a purity of 99.99% (HPLC area%).

### Comparative example 3:

Tadalafil (1g) (99% purity) was suspended in 2-propanol (200mL) and suspension was heated up to reflux temperature until the material was dissolved. The obtained solution was then hot filtrated and cooled to about 10°C. The crystallized tadalafil was centrifuged and dried in an oven at temperature up to 70°C.

## Claims

1. A process for preparing tadalafil which comprises the following steps:
(i) suspending D-tryptophan or a salt thereof or an ester of D-tryptophan with R-OH or a salt thereof and piperonal under acidic conditions in an organic solvent and heating the suspension in a closed reaction vessel above the reflux temperature and at pressure of at least 0.1MPa, or heating the suspension in a closed reaction vessel at an increased pressure at a temperature above the boiling point of the employed organic solvent at normal atmospheric pressure,
(ii) chloroacylation of the "in situ" formed intermediate, preferably intermediate A which is a mixture of cis and trans isomer, with formula, where R is C₁-C₆ alkyl, preferably Me, Et, Pr, *i*Pr, *t*Bu, cyclohexyl, more preferably Me, and HX is organic or inorganic acid, preferably HCl 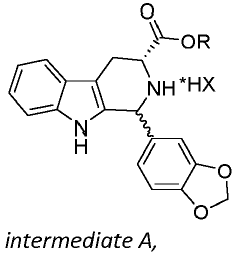 in the presence of a base,
(iii) isolation of the obtained intermediate, preferably intermediate B, with formula where R is C₁-C₆ alkyl, preferably Me, Et, Pr, *i*Pr, *t*Bu, cyclohexyl, more preferably Me 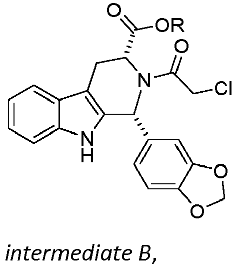 and converting it to tadalafil via cyclisation with methylamine in the mixture of organic solvent with water, in a closed reaction vessel at temperature above the reflux temperature and at pressure of at least 0.1MPa, or in a closed vessel at an increased internal pressure and at a temperature above the boiling point of the employed solvent at normal atmospheric pressure,
(iv) precipitation and isolation of the product,
(v) optionally performing re-crystallization under increased pressure.

2. A process according to claim 1, wherein the solvent used in step (iii) and/or step (iv) and step (v) is selected from alcohol such as ethanol, methanol, n-propanol, and 2-propanol, preferably 2-propanol, optionally in combination with water.

3. A process according to claim 2, wherein step (iii) and/or step (iv) and step (v) is carried out in a mixture of 2-propanol and water.

4. A process according to any of the preceding claims wherein step (i), step (iii) and/or (v) are carried out at pressure in the range of 0.1MPa to 0.4MPa.

## Patentansprüche

1. Verfahren zur Herstellung von Tadalafil, das die nachstehenden Schritte umfasst:
(i) Suspendieren von D-Tryptophan oder einem Salz davon oder einem Ester von D-Tryptophan mit R-OH oder einem Salz davon und Piperonal unter sauren Bedingungen in einem organischen Lösungsmittel und Erwärmen der Suspension in einem geschlossenen Reaktionsgefäss oberhalb der Rückflusstemperatur und bei einem Druck von zumindest 0,1 MPa oder Erwärmen der Suspension in einem geschlossenen Reaktionsgefäss bei erhöhtem Druck bei einer Temperatur oberhalb des Siedepunktes des eingesetzten organischen Lösungsmittels bei normalem atmosphärischen Druck,
(ii) Chloracylierung des "in situ" gebildeten Intermediats, vorzugsweise Intermediat A, das eine Mischung von cis- und trans-Isomeren ist, mit der Formel, worin R C₁-₆-Alkyl, vorzugsweise Me, Et, Pr, iPr, tBu, Cyclohexyl, stärker bevorzugt Me, ist und HX organische oder anorganische Säure, vorzugsweise HCl, ist 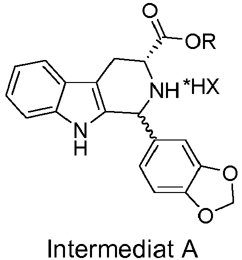 in Gegenwart einer Base,
(iii) Isolieren des erhaltenen Intermediats, vorzugsweise Intermediat B, mit der Formel, worin R C₁-₆-Alkyl, vorzugsweise Me, Et, Pr, iPr, tBu, Cyclohexyl, stärker bevorzugt Me, ist 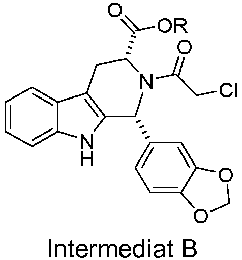 und Umwandeln zu Tadalafil durch Cyclisierung mit Methylamin in einer Mischung aus organischem Lösungsmittel mit Wasser in einem geschlossenen Reaktionsgefäss bei einer Temperatur oberhalb der Rückflusstemperatur und bei einem Druck von zumindest 0,1 MPa oder in einem geschlossenen Gefäss bei einem erhöhten Innendruck und bei einer Temperatur oberhalb des Siedepunktes des eingesetzten Lösungsmittels bei normalem atmosphärischen Druck,
(iv) Ausfällen und Isolieren des Produkts,
(v) gegebenenfalls Durchführen einer Umkristallisation unter erhöhtem Druck.

2. Verfahren gemäss Anspruch 1, wobei das in Schritt (iii) und/oder Schritt (iv) und Schritt (v) verwendete Lösungsmittel aus Alkohol, wie Ethanol, Methanol, n-Propanol und 2-Propanol, vorzugsweise 2-Propanol, gegebenenfalls in Kombination mit Wasser, ausgewählt ist.

3. Verfahren gemäss Anspruch 2, wobei Schritt (iii) und/oder Schritt (iv) und Schritt (v) in einer Mischung von 2-Propanol und Wasser durchgeführt wird.

4. Verfahren gemäss einem der vorhergehenden Ansprüche, wobei Schritt (i), Schritt (iii) und/oder (v) bei einem Druck im Bereich von 0,1 MPa bis 0,4 MPa durchgeführt werden.

## Revendications

1. Procédé de préparation de tadalafil qui comprend les étapes suivantes :
(i) mise en suspension de D-tryptophane ou d'un sel de celui-ci ou d'un ester de D-tryptophane avec du R-OH ou un de ses sels et du pipéronal dans des conditions acides dans un solvant organique et chauffage de la suspension dans un récipient réactionnel fermé au-dessus de la température de reflux et à une pression d'au moins 0,1 MPa, ou chauffage de la suspension dans un récipient réactionnel fermé à une pression accrue à une température supérieure au point d'ébullition du solvant organique employé à pression atmosphérique normale,
(ii) chloroacylation de l'intermédiaire formé « in situ », de préférence de l'intermédiaire (A) qui est un mélange d'isomères cis et trans avec une formule où R est un alkyle en C₁-C₆, de préférence Me, Et, Pr, *i*Pr, *t*Bu, cyclohexyle, mieux encore Me, et HX est un acide organique ou inorganique, de préférence HCI, 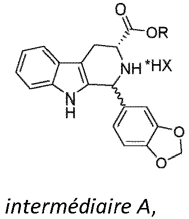 en présence d'une base,
(iii) isolement de l'intermédiaire obtenu, de préférence l'intermédiaire B avec une formule où R est un alkyle en C₁-C₆, de préférence Me, Et, Pr, *i*Pr, *t*Bu, cyclohexyle, mieux encore Me : 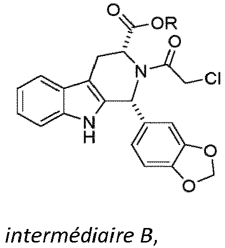 et sa conversion en tadalafil par cyclisation avec de la méthylamine dans le mélange de solvant organique avec de l'eau, dans un récipient réactionnel fermé à une température au-dessus de la température de reflux et à une pression d'au moins 0,1 MPa, ou dans un récipient fermé à une pression interne accrue et à une température supérieure au point d'ébullition du solvant utilisé à pression atmosphérique normale,
(iv) précipitation et isolement du produit,
(v) réalisation éventuelle d'une recristallisation sous pression accrue.

2. Procédé selon la revendication 1, dans lequel le solvant utilisé à l'étape (iii) et/ou à l'étape (iv) et à l'étape (v) est choisi parmi un alcool tel que l'éthanol, le méthanol, le n-propanol, et le 2-propanol, de préférence le 2-propanol, éventuellement en combinaison avec de l'eau.

3. Procédé selon la revendication 2, dans lequel l'étape (iii) et/ou l'étape (iv) et l'étape (v) est ou sont effectuées dans un mélange de 2-propanol et d'eau.

4. Procédé selon l'une quelconque des revendications précédentes, dans lequel l'étape (i), l'étape (iii) et/ou l'étape (v) est ou sont effectuées à une pression dans la plage de 0,1 MPa à 0,4 MPa.
